# DEMANDE DE BREVET EUROPEEN

(11) **EP 2 556 845 A1**
(43) Date de publication de la demande: **13.02.2013**
(21) Numéro de dépôt: 12191516.9
(22) Date de dépôt: 29.08.2008
(51) Int. Cl.: A61L 31/14, A61L 24/04, A61L 31/10, A61L 24/06, A61L 24/00, A61F 2/00

(54) **Implant textile, notamment pour la refection des hernies**

(30) Priorité: 07.09.2007 FR 0757427
(62) Demande divisionnaire de: 08829748.6
(71) Demandeur: Cousin Biotech, 59117 Wervicq Sud (FR)
(72) Inventeur: Solecki, Gilles, 59390 Lannoy (FR)
(74) Mandataire: Balesta, Pierre

(57) **Abrégé**

L'invention concerne un implant textile (1), notamment pour la réfection des hernies, comprenant une pièce textile (2) dont une première face (2a) est revêtue totalement ou partiellement d'une composition bio-adhésive (3), qui est hydrosoluble et absorbable et qui comprend au moins un polymère bio-adhésif, qui est hydrosoluble et absorbable, dont la fonction adhésive est activable en milieu humide ou mouillé. De manière caractéristique, ladite composition bio-adhésive (3) comprend moins de 4% en poids de plastifiant.

## Description

La présente invention est dans le domaine technique des implants textiles, notamment pour la réfection des hernies, comprenant une pièce textile dont une première face est revêtue totalement ou partiellement d'une composition bio-adhésive qui est hydrosoluble et absorbable, et comprend au moins un polymère bio-adhésif, hydrosoluble et absorbable.

Habituellement, une pièce textile pour la réfection pariétale est fixée sur la zone anatomique à renforcer par des agrafes afin de garantir sa fixation durable sur les tissus humains notamment face aux contraintes mécaniques exercées par les muscles abdominaux, viscères et autres organes susceptibles de bouger la pièce textile de la disposition dans laquelle le praticien l'a implantée.

Ces agrafes sont traumatiques puisque souvent des douleurs postopératoires sont observées, notamment lorsque qu'une terminaison nerveuse est pincée. De plus suturer au moyen d'agrafes est une opération longue et fastidieuse.

Pour répondre à ces inconvénients, des adhésifs chirurgicaux, aptes à faire adhérer la pièce textile aux tissus humains, ont été développés tels que les colles à base de fibrine ou de cyanoacrylate.

Les colles de fibrine sont des colles tissulaires biologiques dérivées du plasma humain. Ces colles renferment les composantes nécessaires à la dernière étape de la coagulation et sont utilisées habituellement au cours d'interventions chirurgicales afin d'arrêter le saignement et pour favoriser la guérison des plaies.

Les colles de fibrine ne sont pas prêtes à l'emploi puisque la formulation doit être préparée en bloc opératoire pour éviter les risques de contamination. Une telle préparation est en outre complexe et longue.

Les colles de fibrine ont un faible pouvoir adhésif de manière générale, et particulièrement comparé aux adhésifs à base de cyanoacrylate.

Les colles de cyanoacrylate ont un fort pouvoir d'adhésion sur les tissus humains mais nécrosent ces derniers, voire les brûlent par réaction exothermique. De plus, les colles de cyanoacrylate durcissent très rapidement, ce qui empêche le repositionnement de la pièce textile sur la zone à traiter par le praticien. Enfin la bio-compatibilité de ces colles n'est pas prouvée. En effet, la réaction de durcissement exothermique ayant lieu au contact des tissus humains dégage des substances toxiques.

Le document FR 2.863.502, au nom du demandeur, décrit un implant chirurgical comprenant un textile et un polymère biocompatible, le polymère étant hydrosoluble et possédant une aptitude à faire adhérer l'implant sur les tissus humains sous l'action conjuguée d'une force de pression et de molécules d'eau. Parmi les polymères cités, se trouvent notamment la polyvinylpyrrolidone (PVP) et la carboxyméthylcellulose (CMC), dont les propriétés adhésives peuvent éventuellement être ajustées par ajout de polyéthylène glycol (PEG), et plus particulièrement dans un ratio de 64% en poids de PVP pour 36% en poids de PEG.

Or, l'implant textile décrit dans FR 2.863.502 ne donne pas totalement satisfaction. En effet, le demandeur s'est aperçu qu'en bloc opératoire, au moment de la pose dudit implant textile sur les tissus humains, la colle réagissait à tout contact dès l'ouverture de son emballage et donc avant même qu'elle n'ait été activée par le milieu humide des tissus. Il en résultait une perte importante de colle sur les gants du praticien et à l'intérieur de l'emballage, et par conséquent une baisse de résultats en terme de fixation durable de l'implant sur les tissus et de facilité de repositionnement. En pratique une quantité de colle insuffisante subsistait sur l'implant. De plus, lorsque l'implant textile devait être inséré dans un trocart, enroulé sur lui-même, la composition bio-adhésive étant collante bien qu'elle n'ait pas été activée par le milieu humide des tissus, une partie de cette colle se retrouvait sur les deux faces de l'implant textile et risquait alors d'adhérer sur la paroi opposée à la zone à renforcer.

Par ailleurs, il a été remarqué que la colle coulait directement dans le sachet d'emballage suite à l'étape de stérilisation, notamment à l'oxyde d'éthylène, de l'implant au cours de laquelle une élévation de température a lieu. Outre l'aspect inesthétique de telles coulures, cela induit un manque de confiance chez le praticien.

Suite à des essais sur des animaux, il a été observé que l'implant textile après quelques jours d'implantation avait tendance à s'écrouler sur lui-même et n'avait alors plus aucune efficacité. De plus, le pouvoir adhésif n'était pas suffisant pour que la fibrose ait le temps de se développer et de fixer l'implant textile définitivement de sorte que ce dernier avait tendance à bouger de la disposition dans laquelle le praticien l'avait implanté.

La présente invention a pour objet un implant textile, notamment pour la réfection des hernies, amélioré et répondant aux problèmes précités, qui de manière connue comprend une pièce textile dont une première face est revêtue totalement ou partiellement d'une composition bio-adhésive, qui est hydrosoluble et absorbable et qui comprend au moins un polymère bio-adhésif, qui est hydrosoluble et absorbable, dont la fonction adhésive est activable en milieu humide ou mouillé. De manière caractéristique, ladite composition bio-adhésive comprend moins de 4% en poids de plastifiant.

On entend par plastifiant toute substance, autre que des molécules d'eau, qui est apte notamment à diminuer la température de transition vitreuse (Tg) dudit polymère bio-adhésif. En effet, lors des étapes de fabrication, de stérilisation, et de stockage -des molécules d'eau pouvant traverser le sachet d'emballage- de l'implant textile, des molécules d'eau peuvent être présentes dans la composition bio-adhésive et agir tels que des agents plastifiants.

Le demandeur s'est aperçu, contrairement à ce que l'état de la technique préconise, et suite à de nombreux essais en laboratoire effectuées sur des porcs et menés selon les standards GLP (Good Laboratory Practice), qu'une sélection d'une proportion en plastifiant inférieure à 4% de la composition bio-adhésive était suffisante pour abaisser la Tg du polymère bio-adhésif sans qu'une augmentation de la température ambiante -par exemple atteignant environ 60°C pour la stérilisation à l'oxyde d'éthylène- n'atteigne la température de fusion dudit polymère bio-adhésif et évite donc que la composition bio-adhésive ne coule dans le sachet d'emballage de l'implant textile. De plus, la composition bio-adhésive ne colle pas aux gants du praticien sans avoir été préalablement activée par un milieu humide ou mouillé, ce qui facilite sa manipulation par le praticien. La composition bio-adhésive est suffisamment rigide pour procurer une bonne mémoire de forme à l'implant textile et faciliter son déploiement lors de sa sortie du trocart sur les tissus humains et épouser parfaitement la zone à renforcer.

Avantageusement, la composition bio-adhésive est absorbable, ce qui signifie qu'elle est dissoute progressivement par le milieu humide ou mouillé des tissus humains contenant en quantité suffisante des molécules d'eau puis éliminée naturellement par l'organisme. Ainsi, la composition bio-adhésive joue son rôle d'adhésif repositionnable une fois activé en milieu humide ou mouillé sur les tissus, maintient la pièce textile sur lesdits tissus dans la position dans laquelle le praticien l'a placée sans migrer et ce suffisamment longtemps pour laisser la fibrose et les tissus conjonctifs se développer pour fixer définitivement la pièce textile. La pièce textile assure alors à elle seule le rôle d'agent de renfort mécanique. Il n'est pas nécessaire d'employer des agrafes. De plus, la colle employée étant éliminée par l'organisme, la quantité d'agents étrangers à l'organisme est diminuée ce qui limite les risques de complications et améliore la tolérance de l'organisme vis-à-vis dudit implant textile.

L'implant textile selon la présente invention peut être utilisé pour la réfection notamment des hernies inguinales, crurales et ombilicales.

De préférence, la pièce textile est un tricot, du type chaîne ou raschel, à base de monofilaments, de diamètre compris dans l'intervalle [0.0 mm ; 0.3 mm] choisi parmi les polymères suivants : polypropylène, polyamide ou polyester. De préférence, la pièce textile est ajourée, avec des jours de l'ordre du millimètre pour favoriser l'accroche des tissus conjonctifs développés suite à la fibrose sur les mailles délimitant les jours de ladite pièce textile.

De préférence, la composition bio-adhésive comprend un seul polymère bio-adhésif, hydrosoluble et absorbable.

Dans une variante de réalisation, ladite composition bio-adhésive comprend moins de 2% en poids de plastifiant, de préférence choisi parmi les polyalcools, notamment le polyéthylène glycol (PEG).

Le demandeur s'est aperçu qu'une quantité inférieure à 2% de plastifiant procurait des résultats satisfaisants et permettait encore de diminuer la quantité d'agents étrangers dans l'organisme. De plus, la Tg du polymère bio-adhésif étant moins abaissée, la composition bio-adhésive a moins de risque de couler et d'être collante sans avoir été activée. Les polyalcools sont préférés en tant que plastifiant, notamment le PEG, et joue également le rôle d'agent solubilisant du polymère bio-adhésif lors de la préparation de la composition bio-adhésive.

Dans une variante de réalisation, le ou chaque plastifiant a une masse moléculaire moyenne en poids Mw comprise entre 100 g/mol et 700 g/mol.

Dans une variante de réalisation, le ou chaque un polymère bio-adhésif est choisi parmi les polymères suivants : carboxyméthylcellulose (CMC), polyvinylpyrrolidone (PVP), polyacryliques et de préférence la polyvinylpyrrolidone (PVP).

Dans une variante de réalisation, le ou chaque un polymère bio-adhésif a une masse moléculaire moyenne en poids Mw comprise entre 44 000 g/mol et 2.10⁶ g/mol, notamment de la polyvinylpyrrolidone (PVP).

Le demandeur s'est aperçu que si la masse moléculaire moyenne en poids du polymère bio-adhésif est trop faible, la composition bio-adhésive se dissout intégralement au premier contact des tissus humains. Le praticien ne peut plus alors repositionner l'implant textile sur les tissus puisqu'il n'y a pratiquement plus de colle. Le pouvoir adhésif étant très diminué, l'implant textile migre et ne reste pas en place correctement sur la zone à renforcer.

Une masse moléculaire moyenne en poids comprise en 44 000 g/mol et 2.10⁶ g/mol permet de pallier les problèmes précités. De préférence Mw est comprise dans l'intervalle [1.10⁶ ; 2.10⁶] g/mol ce qui donne des résultats améliorés en terme de pouvoir adhésif pour le repositionnement par le praticien de l'implant textile et le maintien en place de la pièce textile suffisamment longtemps pour laisser la fibrose se développer. Dans le cas de la réfection des hernies, le demandeur s'est aperçu que plus la masse moléculaire moyenne en poids du polymère bio-adhésif est élevée, plus le pouvoir adhésif de ladite composition bio-adhésive est fort.

Dans une variante de réalisation, sur la première face de ladite pièce textile, la composition bio-adhésive est disposée selon des motifs distants les uns des autres d'au moins 1 mm, de préférence d'au moins 1,5 mm.

Etant donné que la masse moléculaire moyenne en poids est élevée et que la quantité de plastifiant doit être inférieure à 4% en poids de la composition bio-adhésive, pour que l'implant textile ne colle pas sans avoir été activé et/ou ne coule dans le sachet d'emballage, la composition bio-adhésive est assez rigide (notamment pour une quantité de plastifiant inférieur à 2% et particulièrement pour environ 1%) et à tendance à se fissurer lors de l'enroulement de l'implant textile sur lui-même pour son placement dans un trocart, le praticien lui fait subir un rayon de courbure trop faible, par exemple supérieure à 30%. En espaçant les motifs de la composition bio-adhésive d'au moins 1 mm, et de préférence d'au moins 1,5 mm, le demandeur s'est aperçu qu'il est possible d'enrouler l'implant textile sur lui-même de sorte qu'il soit introduit dans un trocart ayant un diamètre de 10 mm ou 12 mm sans que la composition bio-adhésive sèche ne se fissure.

Dans une variante de réalisation, lesdits motifs sont des bandes parallèles, de préférence ayant une largeur de l'ordre de 4 mm et distantes de l'ordre de 2 mm.

L'implant textile est ainsi avantageusement enroulé sur lui-même en rabattant les bandes parallèles les unes contre les autres. La zone de pliage entre deux bandes correspond de préférence à la zone exempte de composition bio-adhésive séparant lesdites bandes, dans ce cas particulier avec une largeur de l'ordre de 2 mm.

Dans une variante de réalisation, lesdits motifs sont dits chiraux, n'étant pas superposables à leur image dans un miroir, de préférence ayant une forme de S ayant une largeur de l'ordre de 5 à 8 mm, une hauteur de l'ordre de 20 mm et distants de l'ordre de 1,5 mm.

La composition bio-adhésive, selon la nature du polymère bio-adhésif et du plastifiant employés, notamment s'agissant de la PVP avec du PEG, est le plus souvent transparente. Le demandeur s'est aperçu qu'une fois la composition bio-adhésive appliquée sur une pièce textile ajourée, par exemple un tricot à partir de monofilaments en polypropylène (PP), le praticien ne parvenait pas facilement en bloc opératoire à distinguer la première face ayant une fonction adhésive activable de la second face. Avantageusement, la disposition consistant à ce que la composition bio-adhésive soit enduite sur la première face selon des motifs chiraux pallie le problème précité en permettant au praticien d'identifier facilement et rapidement la première face de l'implant textile et ce sans mouiller ladite première face avant l'implantation.

Dans une variante de réalisation, la masse surfacique de la pièce textile est comprise dans l'intervalle [15 ; 200] g/m², de préférence dans l'intervalle [30 ; 100] g/m².

De préférence, la pièce textile est un tricot du type chaîne ou rachel comprenant des monofilaments en polypropylène, et ayant des jours de l'ordre du millimètre, lesquels facilitent l'accroche des tissus conjonctifs développés suite à la fibrose à ladite pièce textile.

La présente invention a pour objet selon un deuxième aspect un implant textile, notamment pour la réfection des hernies en intra-abdominale extra-péritonéale, selon l'une quelconques des variantes de réalisation décrites ci-dessus, comprenant de manière caractéristique une composition bio-adhésive dont la masse surfacique est supérieure ou égale à trois fois la masse surfacique de la pièce textile.

Bien que dans la conception d'un implant, le but soit de minimiser la quantité de substances étrangères dans l'organisme, le demandeur s'est aperçu que la quantité de composition bio-adhésive nécessaire pour éviter que l'implant textile ne migre une fois implanté devait être au moins trois fois supérieure à la masse surfacique de la pièce textile lorsque l'implant textile est disposé en intra-abdominale extra-péritonéale. Une explication, non exhaustive, serait que l'implant textile étant disposé contre la paroi musculaire, cette dernière subit beaucoup de contraintes mécaniques lesquelles pourraient déplacer l'implant textile de sa position initiale d'implantation bien que le péritoine protège l'implant textile des viscères.

L'implant textile doit répondre notamment à la norme ISO 10993 évaluant la biocompatibilité et la cytotoxicité subcronique et à des tests de sensibilisation. Ces évaluations permettent de s'assurer que l'organisme éliminera parfaitement le polymère bio-adhésif et le plastifiant et tolérera la pièce textile. En l'occurrence, une quantité importante de composition bio-adhésive a été testée comme parfaitement tolérée et absorbée chez l'animal au bout d'environ 28 jours.

Dans une variante de réalisation, la masse surfacique de ladite composition bio-adhésive est comprise dans l'intervalle [45 ; 600] g/m², de préférence dans l'intervalle [90 ; 300] g/m².

La masse surfacique de la pièce textile est alors comprise dans l'intervalle [15 ; 200] g/m², et de préférence dans l'intervalle [30 ; 100] g/m².

La présente invention a pour objet, selon un troisième aspect, un implant textile, notamment pour la réfection des hernies en intra-abdominale intra-péritonéale, selon l'une quelconque des variantes de réalisation décrites ci-dessus, dont de manière caractéristique :
- la seconde face de la pièce textile est revêtue totalement ou partiellement d'un matériau polymère ayant un coefficient de frottement inférieur à 0,1,
- et la masse surfacique de ladite composition bio-adhésive est au moins égale au tiers de la masse surfacique de la pièce textile.

L'implant textile, étant disposé de préférence en intra-abdominale intra-péritonéale, se retrouve entre le péritoine et les viscères. Ladite seconde face étant de préférence orientée vers les viscères, ledit matériau polymère ayant un faible coefficient de frottement évite que celles-ci n'adhèrent à la seconde face et donc à l'implant textile. La première face ayant une fonction adhésive activable est destinée à être appliquée sur à la zone à renforcer.

Contrairement à l'implantation en intra-abdominale extra-péritonéale, le demandeur s'est aperçu qu'une quantité de composition bio-adhésive égale ou un peu supérieure au tiers de la masse surfacique de la pièce textile est suffisante. Une explication, non exhaustive, est que les viscères n'exercent pas autant de frottements, comme l'homme du métier pourrait le penser, mais au contraire exerce une pression contre la seconde face favorisant le maintien de l'implant textile contre le péritoine. Par ailleurs, le péritoine serait une région soumise à moins de contraintes mécaniques que la paroi abdominale.

Le matériau polymère à faible coefficient de frottement peut être un polymère fluoré ou un polymère à base de diméthylesiloxane (silicone).

Dans une variante de réalisation, ladite seconde face est revêtue d'un matériau polymère fluoré, de préférence du ePTFE (polytétrafluoroéthylène expansé), notamment sous la forme d'un film.

Dans une variante de réalisation, ladite masse surfacique de la composition bio-adhésive est comprise dans l'intervalle [5 ; 70] g/m², de préférence dans l'intervalle [10 ; 40] g/m².

La masse surfacique de la pièce textile est alors comprise dans l'intervalle [15 ; 200] g/m², de préférence dans l'intervalle [30 ; 100] g/m².

La présente invention sera mieux comprise à la lecture des exemples de réalisation ci-après, cités à titre non limitatif, et illustrés dans les figures suivantes, annexées à la présente, et dans lesquelles :
- la figure 1 est une représentation schématique d'une coupe transversale d'un premier exemple d'implant textile selon la présente invention schématisée dans l'organisme pour la réfection des hernies en intra-abdominale extra-péritonéale,
- la figure 2 est une représentation schématique d'une première variante de la première face de l'implant textile représenté à la figure 1,
- la figure 3 est une représentation schématique d'une seconde variante de la première face d'un implant textile selon la présente invention,
- la figure 4 est une représentation schématique d'une coupe transversale d'un deuxième exemple d'implant textile selon la présente invention schématisée dans l'organisme pour la réfection des hernies en intra-abdominale intra-péritonéale.

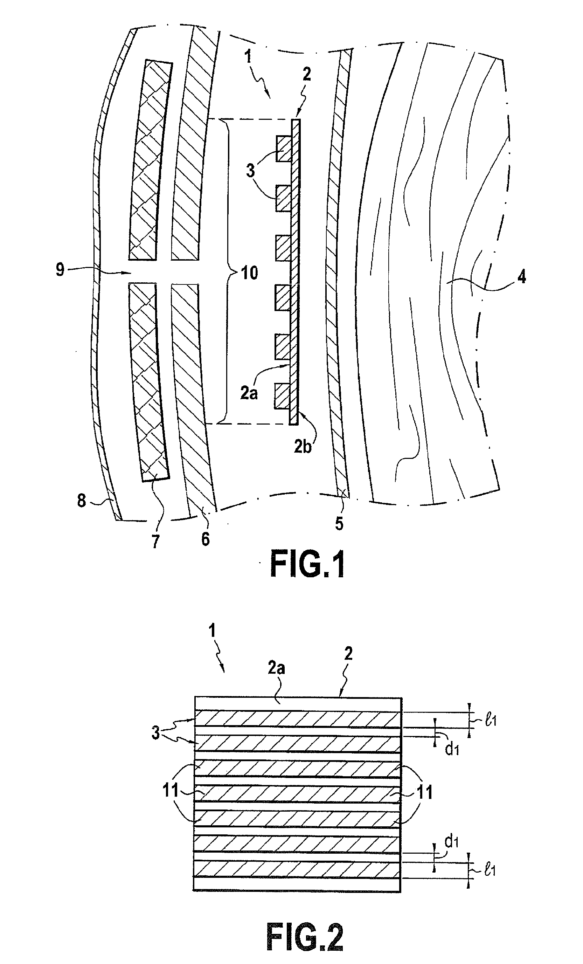

L'implant textile 1 représenté aux figures 1 et 2 comporte une pièce textile 2 ayant une première face 2a et une seconde face 2b. La première face 2a est revêtue partiellement d'une composition bio-adhésive 3 dont la fonction adhésive est activable au contact du milieu humide des tissus. L'implant textile 1 est implanté en intra-abdominale extra-péritonéale, il est donc disposé entre les viscères 4 et le péritoine 5 d'un côté et la paroi abdominale 6, une couche de tissu adipeux 7 et la peau 8 de l'autre côté.

Sur la figure 1, la première face 2a de l'implant textile 1 est disposée au regard de l'orifice herniaire ou de l'éventration 9, obturant la couche de tissu adipeux 7 et le péritoine 6, après que le sac herniaire ait été disséqué et repoussé (non représenté). La composition bio-adhésive 3 est hydrosoluble et absorbable et comprend de préférence un seul polymère bio-adhésif qui est hydrosoluble et absorbable et dont la fonction adhésive est activable au contact du milieu humide ou mouillé des tissus. Dans ce cas particulier, le polymère bio-adhésif est de la polyvinylpirrolidone (PVP), de préférence celui commercialisé sous la marque Kollidon 90F ® par BASF et ayant une masse moléculaire moyenne en poids Mw comprise entre 1,0. 10⁶ g/mol à 1,5.10⁶ g/mol.

La composition bio-adhésive comprend moins de 2% en poids de plastifiant, de préférence de l'ordre de 1% en poids d'un polyalcool, de préférence du polyéthylène glycol (PEG). Le PEG préféré a une masse moléculaire moyenne en poids comprise entre 100 g/mol et 700 g/mol. La pièce textile 2 est de préférence un tricot, du type chaîne ou raschel, à base de mono-filaments en polypropylène ayant un diamètre compris dans l'intervalle [0.0 mm ; 0.3 mm], et présentant des ouvertures ou jours de l'ordre du millimètre, de préférence de l'ordre de 2 mm * 3 mm ou de 3 mm * 3 mm. Ces ouvertures facilitent la fixation définitive de la pièce textile 2 par développement des tissus conjonctifs à travers celles-ci. La pièce textile 2 a une masse surfacique comprise dans l'intervalle [15 ; 200] g/m², et de préférence comprise dans l'intervalle [30 ; 100] g/m².

La masse surfacique de la composition bio-adhésive 3 est comprise dans l'intervalle [45 ; 600] g/m², et de préférence dans l'intervalle [90 ; 300] g/m². Dans un exemple précis, la pièce textile 2 a une masse surfacique de l'ordre de 30 g/m² et la masse surfacique de la composition bio-adhésive est comprise entre 115 g/m² et 210 g/m², et vaut de préférence environ 120 g/m². La masse surfacique de la composition bio-adhésive 3 est au moins trois fois supérieure à la masse surfacique de la pièce textile 2, et dans cet exemple précis de l'ordre de 4 fois la masse surfacique de la pièce textile 2.

Sur la première face 2a, la composition bio-adhésive 3 est disposée selon des motifs 11 distants les uns des autres d'une distance d1 d'au moins 1 mm, dans cet exemple précis d1 vaut 2 mm. Les motifs 11 sont des bandes parallèles ayant une largeur l1 de préférence de 4 mm. Les motifs 11 permettent avantageusement d'enrouler facilement l'implant textile 1 sur lui-même dans le sens transversal à la direction longitudinale des dites bandes, sans que la composition bio-adhésive 3 ne se fissure. En effet, le pouvoir adhésif de la composition bio-adhésive étant fort, la masse moléculaire moyenne en poids du polymère bio-adhésive sélectionné est importante, et donc la composition bio-adhésive est relativement rigide, et ce d'autant plus que la quantité de plastifiant est faible. L'agencement de la composition bio-adhésive 3 selon des motifs 11 espacés, confère davantage de souplesse à l'implant textile 1, notamment pour l'enrouler sur lui-même.

En fonctionnement, l'implant textile 1 est de préférence introduit à l'aide d'un trocart, ayant un diamètre de l'ordre de 10-12 mm, à l'intérieur duquel l'implant textile 1 est disposé enroulé sur lui-même, puis poussé à travers le trocart dans la zone d'implantation. La composition bio-adhésive 3 sèche est suffisamment rigide pour conférer à l'implant textile 1 une bonne mémoire de forme, ce qui permet à ce dernier de se déployer facilement et totalement en sortie du trocart. La composition bio-adhésive 3 conserve cependant une relative souplesse permettant à ce que l'implant textile 1 épouse parfaitement la zone 10 du péritoine 6 à renforcer selon toute la surface de la première face 2a.

Au contact du milieu humide voire mouillé des tissus contenant de l'eau, la fonction adhésive de la composition bio-adhésive 3 est activée, et l'implant textile 1 adhère immédiatement à la zone 10. La quantité et le pouvoir adhésif de la composition bio-adhésive 3 sont suffisants pour que le praticien puisse repositionner parfaitement l'implant textile 1 autant de fois qu'il le souhaite, et que l'implant textile 1 reste dans cette position initiale d'implantation sur la zone 10 à renforcer au moins pendant 28 jours, correspondant à la durée moyenne nécessaire pour que la fibrose se développe et maintienne définitivement la pièce textile 2 sur ladite zone 10. De plus, le pouvoir adhésif et la quantité de composition bio-adhésive sont suffisants pour que l'implant textile 1 ne s'écroule pas sur lui-même et que toute la surface de la face 2a reste en contact avec la zone 10 à renforcer suffisamment longtemps pour que la fibrose et le développement des tissus conjonctifs aient lieu et se fixe définitivement la pièce textile 2. Au bout de cette période d'environ 28 jours, la composition bio-adhésive 3 a été totalement absorbée et éliminée naturellement par l'organisme de sorte que seule la pièce textile 2 ayant une masse surfacique faible, de l'ordre de 30 g/m², subsiste et assure le rôle de renfort mécanique de la zone 10.

Le tableau 1 représente les résultats d'une étude GLP (Good Laboratory Practice) ayant pour but d'évaluer les résultats d'implantation de l'implant textile 1 représenté aux figures 1 et 2 sur un animal en utilisant la coelioscopie. Les animaux testés sont des porcs. Des repères, dans ce cas des agrafes, sont placés aux quatre coins de l'implant textile 1 une fois implanté dans le but d'identifier suite à l'explantation si l'implant textile 1 a migré.

**Tableau 1**

| | 1^{er} jour | 7^{ème} jour | 28^{ème} jour |
|---|---|---|---|
| Nombre de porcs testés | Groupe I : 6 | Groupe II : 6 | Groupe III : 6 |
| Nombre d'implants textiles (1) testés | 11 | 12 | 12 |
| Résultats à l'implantation | Dont 11 classés ++++ | Dont 12 classés ++++ | Dont 12 classés ++++ |
| Résultats à l'explantation | Dont 3 classés ++++ | Dont 1 classé ++++ | Dont 3 classés ++++ |
| | Dont 6 classés +++ | Dont 8 classés +++ | Dont 7 classés +++ |
| | Dont 2 classés + | Dont 2 classés ++ | Dont 2 classés ++ |
| | | Dont 1 classé + | |

| | | | |
|---|---|---|---|
| La légende du tableau 1 est la suivante : ++++ : Très bon +++ : Bon ++ : Moyen + : Faible | | | |

Au premier jour, lorsque l'implant textile 1 est implanté, l'évaluation de la migration prend en compte : le maintien d'un positionnement correct de l'implant, la facilité de manipulation et l'effet adhésif immédiat. Au jour de l'explantation, l'évaluation prend en compte : la migration éventuelle de l'implant textile 1, le maintien dans un état déployé et l'effet adhésif à long terme. La technique utilisée imite la réparation d'une hernie par voie laparoscopique. Aucune hernie n'a été recréée en l'occurrence sur les animaux testés. Chaque période : 1^{er} jour, 7^{ème} jour et 28^{ème} jour correspond à un groupe d'animaux respectivement groupe I, groupe II et groupe III. Un résultat faible à l'implantation est caractérisé par un positionnement difficile, un pouvoir adhésif nul, un mauvais déploiement de l'implant textile 1 en sortie du trocart et une migration de sa position initiale. Un résultat très bon à l'implantation est caractérisé par un placement facile, pas de migration de l'implant textile 1 de sa position initiale d'implantation, un pouvoir adhésif fort et un bon déploiement de l'implant textile 1 en sortie du trocart. Un résultat d'implantation qualifié de bon tend vers un résultat très bon et un résultat moyen tend vers un résultat passable.

On remarque que tous les résultats d'implantation sont très bons, ce qui signifie que l'implant textile 1 adhère immédiatement aux tissus humains à renforcer, est facilement repositionnable et possède un pouvoir adhésif fort. Lors de l'explantation des implants textiles 1, environ 80% des implants testés donnent des résultats très bons ou bons, ce qui signifie qu'ils sont toujours en place après 7 ou 28 jours d'implantation, parfaitement contre la paroi à renforcer, sans s'écrouler sur eux-mêmes et surtout qu'ils n'ont pas migré de la position initiale dans laquelle le praticien les avait disposés. Ces résultats sont donc très concluants.

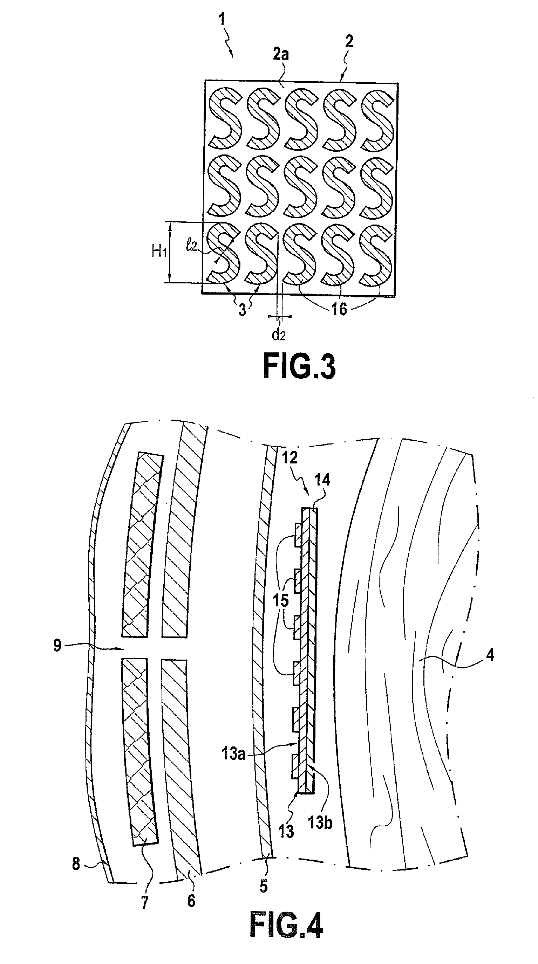

La figure 3 représente une variante des motifs 11 supportés par la première face 2a de l'implant textile 1. En effet, la composition bio-adhésive 3 est revêtue selon des motifs 16 chiraux sur la première face 2a. Les motifs 16 en question ont une forme de S de largeur l2 de l'ordre de 8 mm, de hauteur H1 de 20 mm et distants selon d2 de l'ordre de 1,5 mm. Etant donné que les compositions bio-adhésives sont transparentes et que la pièce textile 2 comporte des ouvertures, il est difficile pour le praticien en bloc opératoire de distinguer la première face 2a de la seconde face 2b. Dans le cas présent, les motifs 16 appliqués sur la première face 2a ont une forme telle qu'ils sont vus sous une configuration différente selon qu'on les regarde depuis la première face 2a ou par transparence depuis la seconde face 2b, ceci permet au praticien d'identifier rapidement, et avec certitude, la première face 2a ayant une fonction adhésive activable.

L'implant textile 12, représenté à la figure 4, est destiné à la réfection des hernies en intra-abdominale intra-péritonéale. Il comprend une pièce textile 13 ayant une première face 13a et une seconde face 13b revêtue totalement d'un matériau polymère 14 ayant un coefficient de frottement inférieur à 0,1, de préférence un polymère fluoré ou un polymère à base de silicone. Dans cet exemple précis le matériau polymère 14 se présente sous la forme d'un film en ePTFE (Polytétrafluoroéhtylène expansé). La première face 13a est revêtue d'une composition bio-adhésive 15 ayant la même formulation que la composition bio-adhésive 3 décrite ci-dessus. La pièce textile 13 est identique à la pièce textile 2. L'implant textile 12 est disposé ici dans l'organisme entre d'un côté les viscères 4 et de l'autre côté le péritoine 5, la paroi abdominale 6, une couche de tissus adipeux 7 et la peau 8, au regard de l'éventration ou de l'orifice herniaire 9 une fois que le sac herniaire a été repoussé (non représenté). La pièce textile 13 a une masse surfacique comprise dans l'intervalle [15 ; 200] g/m², et de préférence dans l'intervalle [30 ; 100] g/m². A titre d'exemple précis, la pièce textile 13 a une masse surfacique de l'ordre de 30 g/m². La composition bio-adhésive 15 a une masse surfacique comprise dans l'intervalle [5 ;70] g/m², et de préférence entre [12 ; 50] g/m². La composition bio-adhésive 15 étant revêtue selon des motifs (non représentés) distants d'au moins 1 mm, l'implant textile 12 conserve sa souplesse, et est apte à être enroulé sur lui-même pour être inséré dans un trocart ayant un diamètre de l'ordre de 10-12 mm.

En fonctionnement, le principe est le même, notamment la fonction adhésive, que pour l'implant textile 1. A la différence cependant que le demandeur a remarqué étonnamment que la quantité de composition bio-adhésive 15 nécessaire pour remplir les mêmes fonctions que celles décrites précédemment pour l'implant textile 1 est considérablement diminuée puisque la masse surfacique de la composition bio-adhésive 15 est égale ou légèrement supérieure à au moins 1/3 de la masse surfacique de la pièce textile 13. Une des explications non exhaustive est que la seconde face 13b étant revêtue d'un matériau polymère 14 ayant un très faible coefficient de frottement, les viscères 4 ne peuvent s'accrocher à l'implant textile 12 notamment par la fibrose de sorte que disposé entre les viscères 4 et la péritoine 5, l'implant textile 12 subirait moins de contraintes mécaniques que l'implant textile 1 entre la paroi abdominale 6 et le péritoine 5.

La composition bio-adhésive 15 peut être revêtue selon des motifs particuliers, chiraux ou non, du moment que ces derniers soient distants d'au moins 1 mm de sorte que la composition bio-adhésive 15 ne soit pas altérée, notamment en se fissurant lors de l'enroulement de l'implant textile 12 sur lui-même. Le praticien n'a ici aucune difficulté en bloc opératoire à distinguer la première face 13a de la seconde face 13b grâce au film en ePTFE 14.

Les composition bio-adhésives 3 et 15 sont préparées à partir d'une dissolution d'au moins un polymère bio-adhésif, notamment de la PVP tel que le Kollidon 90F ® commercialisé par BASF®, avec moins de 4% de plastifiant, de préférence moins de 2% de plastifiant, à titre d'exemple précis environ 1% de plastifiant, tel que du PEG avec une quantité suffisante d'eau distillée. Les proportions en poids sont de l'ordre de 70% en eau distillé, 30% d'au moins un polymère bio-adhésif et hydrosoluble et environ 0,5% de plastifiant. Puis la composition bio-adhésive est appliquée sur la première face de la pièce textile par impression, notamment par impression au pochoir ou au moyen de rouleaux gravés. L'implant textile est ensuite passé à l'étuve de sorte d'évaporer l'eau. Une fois l'implant textile refroidi, il est disposé dans un sachet d'emballage puis stérilisé, de préférence par de l'oxyde d'éthylène. Cette technique de stérilisation connue élève la température dans le sachet d'emballage à environ 60°C et tue les germes. La stérilisation aux rayons gamma ne donne pas satisfaction car le polypropylène est dégradé et le polymère bio-adhésif risquerait de réticuler avec le plastifiant ce qui empêcherait alors sa complète dissolution dans l'organisme et son absorption.

## Revendications

1. Implant textile (1), notamment pour la réfection des hernies comprenant une pièce textile (2) dont une première face (2a) est revêtue totalement ou partiellement d'une composition bio-adhésive (3), qui est hydrosoluble et absorbable et qui comprend au moins un polymère bio-adhésif, qui est hydrosoluble et absorbable, dont la fonction adhésive est activable en milieu humide ou mouillé, **caractérisé en ce que** ladite composition bio-adhésive (3) comprend moins de 2% en poids de plastifiant.

2. Implant textile (1) selon la revendication 1, **caractérisé en ce que** le plastifiant est choisi parmi les polyalcools, notamment le polyéthylène glycol (PEG).

3. Implant textile (1) selon l'une ou l'autre des revendications 1 et 2, **caractérisé en ce que** la composition bio-adhésive (3) comprend au moins un plastifiant ayant une masse moléculaire moyenne en poids Mw comprise entre 100 g/mol et 700 g/mol.

4. Implant textile (1) selon l'une quelconque des revendications 1 à 3, **caractérisé en ce que** le polymère bio-adhésif est choisi parmi les polymères suivants : carboxyméthylcellulose (CMC), polyvinylpyrrolidone (PVP), polyacryliques et de préférence la polyvinylpyrrolidone (PVP).

5. Implant textile (1) selon l'une quelconque des revendications 1 à 4, **caractérisé en ce que** le polymère bio-adhésif a une masse moléculaire moyenne en poids Mw comprise entre 44 000 g/mol et 2.10⁶ g/mol, notamment de la polyvinylpyrrolidone (PVP).

6. Implant textile (1) selon l'une quelconque des revendications 1 à 5, **caractérisé en ce que** sur la première face (2a) de la pièce textile (2), la composition bio-adhésive (3) est disposée selon des motifs (11,16) distants les uns des autres d'au moins 1 mm, de préférence d'au moins 1,5 mm.

7. Implant textile (1) selon la revendication 6, **caractérisé en ce que** les motifs (11) sont des bandes parallèles, de préférence ayant une largeur de l'ordre de 4 mm et distantes de l'ordre de 2 mm.

8. Implant textile (1) selon la revendication 6, **caractérisé en ce que** les motifs (16) sont chiraux n'étant pas superposables à leur image dans un miroir, de préférence ayant une forme de S ayant une largeur de l'ordre de 5 à 8 mm, une hauteur de l'ordre de 20 mm et distants de l'ordre de 1,5 mm.

9. Implant textile (1) selon l'une quelconque des revendications 1 à 8, **caractérisé en ce que** la masse surfacique de la pièce textile (2) est comprise dans l'intervalle [15 ; 200] g/m², de préférence dans l'intervalle [30 ; 100] g/m².

10. Implant textile (1), notamment pour la réfection des hernies en intra-abdominale extra-péritonéale, selon l'une quelconque des revendications 1 à 9, **caractérisé en ce que** la masse surfacique de ladite composition bio-adhésive (3) est supérieure ou égale à trois fois la masse surfacique de la pièce textile (2).

11. Implant textile (1) selon la revendication 10, **caractérisé en ce que** la masse surfacique de la composition bio-adhésive (3) est comprise dans l'intervalle [45 ; 600] g/m², de préférence dans l'intervalle [90 ; 300] g/m².

12. Implant textile (12), notamment pour la réfection des hernies en intra-abdominale intra-péritonéale, selon l'une quelconque des revendications 1 à 9, **caractérisé en ce que** :
- la seconde face (13b) de la pièce textile (3) est revêtue totalement ou partiellement d'un matériau polymère (14) ayant un coefficient de frottement inférieur à 0,1 ;
- et **en ce que** la masse surfacique de ladite composition bio-adhésive (15) est au moins égale ou supérieure au tiers de la masse surfacique de la pièce textile (13).

13. Implant textile (12) selon la revendication 12, **caractérisé en ce que** la seconde face (13b) est revêtue d'un film d'un matériau polymère (14) fluoré, de préférence du ePTFE (polytétrafluoroéthylène expansé), notamment sous la forme d'un film.

14. Implant textile (12) selon l'une ou l'autre des revendications 12 et 13, **caractérisé en ce que** la masse surfacique de la composition bio-adhésive (15) est comprise dans l'intervalle [5 ; 70] g/m², de préférence dans l'intervalle [10 ; 40] g/m².
